**Europäisches Patentamt**

**European Patent Office**

**Office européen des brevets**

(19)

(11) Veröffentlichungsnummer: **0 044 322**
**B1**

(12) **EUROPÄISCHE PATENTSCHRIFT**

(45) Veröffentlichungstag der Patentschrift:
**03.10.84**

(51) Int. Cl.³: **A 61 B 5/02,** F 16 K 25/00

(21) Anmeldenummer: **81900308.8**

(22) Anmeldetag: **27.01.81**

(86) Internationale Anmeldenummer:
**PCT/EP 81/00010**

(87) Internationale Veröffentlichungsnummer:
**WO 81/02096 (06.08.81** Gazette 81/19)

(54) **ABLASSVENTIL FÜR BLUTDRUCKMESSGERÄTE.**

(30) Priorität: **28.01.80 DE 3002907**

(43) Veröffentlichungstag der Anmeldung:
**27.01.82 Patentblatt 82/4**

(45) Bekanntmachung des Hinweises auf die Patenterteilung:
**03.10.84 Patentblatt 84/40**

(84) Benannte Vertragsstaaten:
**FR GB**

(56) Entgegenhaltungen:
**FR - A - 2 284 075**
**FR - A - 2 336 618**
**US - A - 3 693 611**
**US - A - 3 875 961**
**US - A - 4 142 518**

(73) Patentinhaber: **RUDOLF RIESTER GMBH & CO. KG.**
**FABRIK MED. APPARATE, D-7455 Jungingen (DE)**

(72) Erfinder: **RIESTER, Karlheinz, Bruckstrasse 35,**
**D-7455 Jungingen (DE)**

(74) Vertreter: **Zimmermann, Heinz, Dipl.-Ing., Patentanwälte**
**Leinweber & Zimmermann Rosenthal 7/II Aufg.,**
**D-8000 München 2 (DE)**

ACTORUM AG

**Beschreibung**

Die Erfindung bezieht sich auf ein Ablassventil für Blutdruckmessgeräte mit einem Ventilgehäuse, dessen Innenraum mit einer aufblasbaren Manschette und einem Druckmesswerk sowie einem Luftauslass in Verbindung steht, der zu einem auf der Aussenseite des Ventilgehäuses angeordneten ebenen Ventilsitz führt und dort von einer kreisringförmigen, ebenen, gummielastischen Ventilscheibe abgedeckt ist, die mit ihrem Zentraldurchbruch koaxial auf einem neben dem Ventilsitz vorgesehenen zapfenförmigen Führungsteil angeordnet ist und an ihrem Aussenrand mit einem koaxialen, rotationssymmetrischen Betätigungsglied für das Abheben vom Ventilsitz versehen ist.

Ein bekanntes Ablassventil dieser Art (DE-AS 2 558 058) weist einen in der Achse des Ventilgehäuses liegenden Druckraum auf, an dem in einer Achsrichtung das Druckmesswerk und in der gegenüberliegenden Achsrichtung auf übliche Weise über ein Rückschlagventil eine Gummiballpumpe angeschlossen ist. Der Anschluss der aufblasbaren Manschette erfolgt beispielsweise über einen üblicherweise seitlich am Ventilgehäuse angeschlossenen Gummischlauch. Beispielsweise für die Anwendung in Blutdruckmessgeräten muss der Druck im Druckraum über ein Ablassventil abbaubar sein. Hierfür sind beim bekannten Gerät an den Druckraum unter etwa 45° zur Achse geneigte Verbindungsleitungen angeordnet, die in zur Achse des Gerätes koaxialen Ringnuten münden. Diese sind in einer im übrigen ebenen als Ventilsitz dienenden Kreisringfläche angeordnet und hier von der gummielastischen Ventilscheibe abgedeckt. Die Ventilscheibe wird von einer Ventilfeder auf den Ventilsitz gedrückt, die den zapfenförmigen Führungsteil aussen umgibt und an der gummielastischen Scheibe von der vom Ventilsitz abgewandten Seite her angreift. Die Ventilfeder ist als Schraubenfeder ausgebildet. Der Innenrand der Ventilscheibe ist am Ventilgehäuse fest von beiden Seiten her eingespannt, der Aussenrand mit einem ringförmigen Betätigungsglied versehen. Durch Angreifen an diesem Betätigungsglied kann die Ventilscheibe unter lokaler Verformung der Ventilfeder vom Ventilsitz abgehoben werden, was ein bequemes, fein dosierbares Ablassen der komprimierten Luft und damit den für den Verwendungszweck notwendigen Abbau des Drucks ermöglicht.

Das derart ausgebildete Ablassventil ist jedoch herstellungsaufwendig. Es erfordert eine grosse Anzahl von teilweise kompliziert aufgebauten Einzelteilen, zur Achse und einem Winkel verlaufende und entsprechend schwierig herzustellende Bohrungen und insbesondere auch die herkömmliche Ventilfeder.

Aufgabe der Erfindung ist es demgegenüber, das Ablassventil der eingangs genannten Art bei Beibehaltung des guten Funktionsverhaltens herstellungstechnisch zu vereinfachen. Diese Aufgabe wird durch die in den Ansprüchen gekennzeichnete Erfindung gelöst.

Danach ist die ventilfederlose Ventilscheibe auf ihrer vom Luftauslass abgewandt liegenden Seite in einer ebenen kreisringförmigen Abstützfläche abgestützt, deren Durchmesser zumindest nahezu gleich dem einer Kreislinie ist, längs der die Ventilscheibe auf der dem Luftauslass zugewandten Seite am Ventilgehäuse abgestützt ist, wobei der Luftauslass einen kleineren Durchmesser bzw. Achsabstand als die Kreislinie und die Ventilscheibe einen grösseren Durchmesser als die Abstützfläche aufweist.

Man erkennt zunächst, dass die gummielastische Ventilscheibe in ihrem kreisringförmigen achsnahen Bereich mit ihrer ebenen Oberfläche auf einer ebenen Abstützfläche aufliegt, über die sie jedoch nach aussen vorragt. Diese grossflächige Abstützung des Innenbereichs wird noch dadurch unterstützt, dass auf der gegenüberliegenden als Ventilsitz anzusehenden Seite ebenfalls eine Abstützung längs einer Kreislinie erfolgt, deren Durchmesser zumindest nahezu gleich demjenigen der Abstützfläche ist. Die grossflächige Abstützung erzielt so eine verkippungsfreie Lagerung der Ventilscheibe, die so zugleich eine sichere und durchgehende Anlage längs der Kreislinie auf der gegenüberliegenden Seite ist. Die Kreislinie hat selbstverständlich eine gewisse endliche, jedoch überaus geringe Breite. Durch diese Dimensionierung ist es möglich, den Aussenteil der Ventilscheibe mit Hilfe des Betätitungsgliedes geringfügig in Richtung auf die Abstützfläche abzuheben und damit auf der gegenüberliegenden Seite den der Abdichtung dienenden Kreislinienkontakt mit dem Ventilgehäuse ausserhalb des Luftauslases aufzuheben, wodurch die Ablasswirkung erzielt wird. Das kann feindosiert erfolgen. Nach dem Loslassen des Betätigungsgliedes kehrt die Ventilscheibe aufgrund ihrer Gummielastizität und in Anbetracht der Art ihrer Abstützung selbsttätig in den dichtenden Kreislinienkontakt mit dem Ventilgehäuse zurück. Es ist deshalb hier nicht mehr erforderlich, die bisher als unumgänglich notwendig angesehene Ventilfeder zu verwenden. Damit entfällt aber nicht nur die Ventilfeder selbst, sondern überdies auch das für die Ventilfeder bisher erforderliche Gegenlager. Die Ausbildung der Teile wird damit vereinfacht. Auch die Abstützfläche und der Luftauslass lassen sich damit materialsparend und in einfachen Arbeitsgängen herstellen.

Für die Funktion besonders günstig ist eine Dimensionierung derart, dass der Durchmesser der Abstützfläche gleich oder grösser als der der Kreislinie ist und der Durchmesser des Luftauslasses nur ganz geringfügig kleiner als der der Kreislinie. Auf diese Weise liegt einerseits der Kreislinie noch eine die Ventilscheibe von der Gegenseite her beaufschlagende Fläche, nämlich der Aussenrand der Abstützfläche gegenüber. Die Anlage an der Kreislinie kann dann unter guter Dichtwirkung sichergestellt werden und zwar insbesondere dann, wenn die Dicke der Ventilscheibe den Abstand der Kreislinie bzw. des sie bildenden Wulstringes am Ventilgehäuse von der Abstützfläche geringfügig übersteigt. Trotzdem genügen wegen der Nähe des Luftauslasses von dem abstützungslosen Bereich der Ventilscheibe schon geringe Hübe der Ventilscheibe, um die Ablasswirkung sicherzustellen.

Dabei beträgt zweckmässig der Durchmesser der Ventilscheibe mindestens das 1½-fache, vorzugsweise das Doppelte des Durchmessers der Abstützfläche. Es ergibt sich dann für die Auslenkung der Ventilscheibe an ihrem Aussenrand mit Hilfe des Betätigungsgliedes ein genügend grosser Hebel, um den Druckabbau fein dosiert, aber zugleich wirkungsvoll zu ermöglichen. Das ist jedenfalls dann sichergestellt, wenn am Aussenrand der Abstützfläche radial nach aussen eine gegenüber dieser um etwa 45° von der Ventilscheibe weg geneigte konische Ringfläche anschliesst. Diese stellt kein Hindernis für die Auslenkung der Ventilscheibe vom Ventilsitz weg in Richtung der Abstütztfläche dar. Sie vermag zugleich als eine Art Anschlag für die insgesamt zur Verfügung stehende Abhebeamplitude zu dienen. Dadurch werden Überdehnungen der gummielastischen Ventilscheibe vermieden. Der Benutzer erhält ein sicheres und angenehmes Betätigungsgefühl.

Herstellungsmässig besonders günstig ist es, wenn die Ventilscheibe auf der dem Luftauslass zugewandten Seite von einer Luftauslasshülse abgestützt ist, die kreisförmig um die Achse verteilt eine Vielzahl achsparalleler Luftauslasskanäle aufweist, die in der der Ventilscheibe zugewandten Endfläche der Luftauslasshülse innerhalb eines Wulstringes münden, der mit der Ventilscheibe längs der Kreislinie in Berührung steht und eine kreisringförmige Endflächenebene rings umgreift, über die er in Richtung auf die Ventilscheibe geringfügig vorsteht. Diese Ausbildung lässt sich leicht mit Hilfe von Automaten oder im Feinguss herstellen, da sie lediglich rotationssymmetrische bzw. achsparallele Teilformen enthält.

Die Luftauslasshülse kann dabei im Abstand von der vom Wulstring umgriffenen Endflächenebene und parallel zu dieser einen die achsparallelen Luftauslasskanäle verbindenden Ringkanal haben, der auf seiner von der Ventilscheibe abgewandten Seite durch einen ebenfalls achsparallelen Verbindungskanal mit einem zur Achse senkrecht verlaufenden und mit dem Druckraum verbundenen Druckkanal in Verbindung steht. Dabei wird zweckmässig die Luftauslasshülse im Bereich des Ringkanals in einer Durchmesserstufe auf einen zylindrischen Schaft verminderten Durchmessers reduziert, auf den eine mit der Luftauslasshülse im Bereich der Durchmesserstufe unter Zwischenlage einer Dichtung den Ringkanal bildende Zylinderhülse aufgebracht ist, in die seitlich eine die Zylinderhülse durchsetzender und mit dem Druckraum kommunizierender Anschlussstutzen eingeschraubt ist, in den über eine Öffnung der Verbindungskanal mündet. Sämtliche Teile sind somit rotationssymmetrisch bzw. achsparallel oder senkrecht zur Achse ausgebildet, so dass sie ebenfalls auf einfachste Weise und automatisiert in Grossserie hergestellt werden können.

Das gilt auch für das ringförmige Betätigungsglied, wenn dieses im Querschnitt halbkreisförmig ausgebildet wird, wobei der Halbkreis einen Durchmesser aufweist, der höchstens etwa das Dreifache der Dicke der Ventilscheibe beträgt, mit seiner Bogenlinie von der Achse weg nach aussen weist und auf seiner Innenseite eine Ringnut zur Aufnahme des Aussenrandes der Ventilscheibe hat. Eine derartige Ausbildung kann einfach hergestellt werden und ist betätigungsmässig und ästhetisch günstig. Letzteres gilt insbesondere dann, wenn für diesen, wie für alle anderen genannten Teile des Ablassventils rostfreier Edelstahl Verwendung findet.

In der Zeichnung, auf die bezüglich der Offenbarung aller im folgenden nicht näher erläuterten Einzelheiten ausdrücklich verwiesen wird, ist die Erfindung beispielsweise erläutert.

Es zeigen

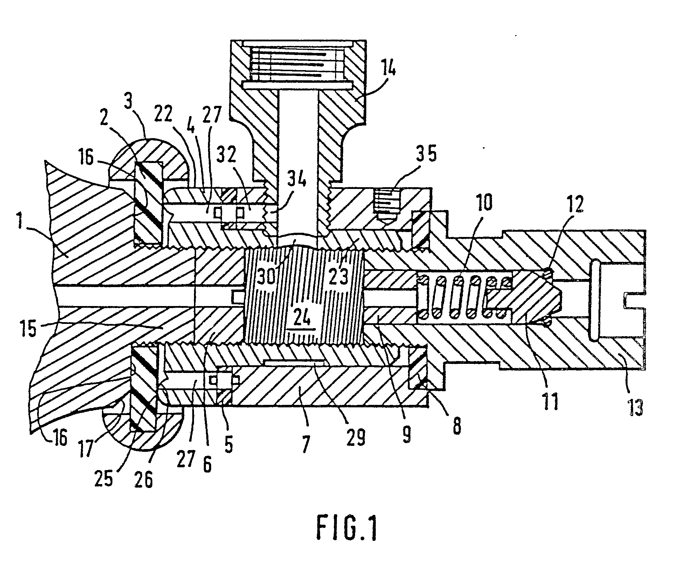

Fig. 1 im Schnitt eine Ausführungsform der Erfindung im zusammengebauten Zustand,

Fig. 2 eine Explosionsansicht der Ausführungsform von Fig. 1.

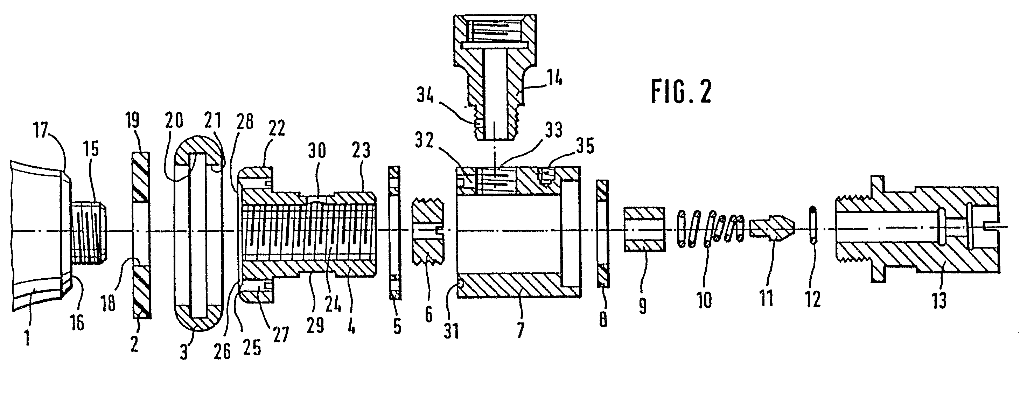

Das Ablassventil besteht aus dem Druckmesswerk 1, einer Ventilscheibe 2, einem die Ventilscheibe umgreifenden Betätigungsglied in Form eines Auslassringes 3, einer Luftauslasshülse 4 mit Dichtung 5 und Fixierstück 6, einem Löffelhalter 7 zur Fixierung des nicht gezeigten Löffels, der als Gegenlager für die Betätigung der ebenfalls nicht gezeigten Gummiballpumpe dient, einer weiteren Dichtung 8, einer Hülse 9, einer Druckfeder 10 zur Beaufschlagung eines Ventilkonus 11 in Richtung auf einen O-Ring 12, sowie einem mit den drei letztgenannten Teilen zusammenwirkenden Rückschlagventil 13. Alle diese Teile sind auf die in Fig. 2 gezeigte Weise axial zusammengesetzt. Quer zur Achse ist ein Anschlussstutzen 14 für eine nicht gezeigte aufblasbare Manschette angebracht. Die Teile sollen nun im einzelnen beschrieben werden.

Das in den Figuren nur mit seinem ablassventilseitigen Ende angedeutete Druckmesswerk 1 weist einen in Achsrichtung vorstehenden zapfenförmigen Führungsteil 15 auf. Der Führungsteil 15 ist zylindrisch ausgebildet und an seinem Aussenumfang mit einem Gewinde versehen. Er steht am Druckmesswerk 1 aus einer Abstützfläche 16 vor, deren Ebene senkrecht zur Achse liegt. Die Abstützfläche 16 ist kreisringförmig und eben. Sie weist keinerlei ihre Abstützfunktion unterbrechenden Einarbeitungen, Mündungen, Nuten oder Senker auf. Am Aussenrand der Abstützfläche 16 schliesst eine gegenüber dieser um etwa 45° vom Führungsteil 15 weggeneigte konische Ringfläche 17 an. Diese stellt die Verbindung von der Abstützfläche 16 zur den Bedürfnissen entsprechend gestalteten Aussenfläche des Druckmesswerks 1 her.

Auf den Führungsteil 15 wird die Einheit aus Ventilscheibe 2 und Auslassring 3 aufgesteckt. Die Ventilscheibe 2 ist ebenfalls kreisringförmig. Ihr Zentraldurchbruch 18 hat einen Innendurchmesser, der dem Aussendurchmesser des Führungsteils 15 entspricht oder diesen etwas übersteigt. Ihr Aussenrand 19 ist in einer Ringnut 20 gelagert, die in die Innenseite des Auslassrings 3 eingearbeitet ist. Dieser Auslassring 3 weist einen etwa halbkreisförmigen Querschnitt auf, mit dem er radial nach aussen gerichtet ist. Sein Innenloch 21 ist zylindrisch und mit der Ringnut 20 versehen. Die achsparallele Bauhöhe des Auslassringes 3 bzw. der Durchmesser seiner nach aussen gewendeten Kreisbogenlinie betragen etwa das Dreifache der Dicke der Ventilscheibe 2. Der Durchmesser der Ventilscheibe beträgt mindestens das 1½-fache, vorzugsweise aber das Doppelte des Durchmessers der Abstützfläche 16.

Die Ventilscheibe 2 besteht aus einem geeigneten gummielastischen Material. Sie muss relativ leicht verformbar sein, andererseits aber nach der Verformung zuverlässig und präzise ihre Ausgangsgestalt wieder annehmen. Die Auslenkung aus der in den Figuren gezeigten Ruhegestalt der Ventilscheibe 2 kann einfach durch Ergreifen des Auslassringes 3 erfolgen.

Sind Ventilscheibe 2 und Auslassring 3 in zusammengebautem Zustand auf den Führungsteil 15 aufgesteckt, so wird auf das Gewinde des Führungsteils 14 die Luftauslasshülse 4 aufgeschraubt, die gegebenenfalls schon vorab auf die unten noch näher zu erläuternde Weise mit den übrigen Teilen zusammengebaut worden ist. Die Luftauslasshülse ist im Querschnitt pilzförmig aufgebaut und besteht aus einem dem Druckmesswerk zugekehrten Kopfflansch 22 und einem Hülsenschaft 23. In ihrem Inneren weist sie den Druckraum 24 auf, der zylinderförmig ausgebildet und mit einem Innengewinde versehen ist, das ein Aufschrauben auf das Aussengewinde des Führungsteils 15 ermöglicht. Der Aussendurchmesser des Kopfflansches 22 unterschreitet den Innendurchmesser des Innenlochs 21.

Der Kopfflansch 22 ist auf seiner der Abstützfläche 16 bzw. der Ventilscheibe 2 zugekehrten Seite mit einem Wulstring 25 versehen. Der Querschnitt des Wulstes ist kreisbogenförmig. Sein Scheitel bildet somit eine Kreislinie längs der die der Luftauslasshülse 4 zugekehrte Seite der Ventilscheibe 2 mit dem Wulstring 25 in Kontakt ist. Diese Kreislinie hat einen Durchmesser, der zumindest nahezu gleich dem der Abstützfläche ist. Der Durchmesser der Abstützfläche kann auch etwas grösser als der dieser Kreislinie sein. Keinesfalls ist er aber beträchtlich grösser. Auf diese Weise wird sichergestellt, dass der Kontakt der Ventilscheibe 2 mit der Luftauslasshülse längs der Kreislinie am Scheitel des Wulstringes 25 durch Bewegen des Auslassrings und Abknicken der gummielastischen Ventilscheibe 2 am Übergang der ebenen Abstützfläche 16 zur konischen Ringfläche 17 leicht aufgehoben werden kann. Der eigentliche Luftauslass schliesst sich an den Wulstring 25 innen, also im achsnäheren Bereich an. Er besteht aus einem nutförmigen Ringkanal 26, in den eine Mehrzahl achsparalleler Luftauslasskanäle 27 mündet, die in dem Bereich des Kopfflansches 22 ausgebildet sind, der radial ausserhalb des Aussenumfangs des Hülsenschaftes 23 liegt. Es sind zumindest zwei einander diametral gegenüberliegende Luftauslasskanäle 27 vorgesehen. Durch die Tatsache, dass die vorhandenen Luftauslasskanäle 27 untereinander durch den Ringkanal 26 verbunden sind kommt es letztlich nicht darauf an, wo die Ventilscheibe 2 mit Hilfe des Auslassrings 3 vom Wulstring 25 abgehoben wird. Für eine gleichmässige Druckverteilung und einen immer gleichen Strömungswiderstand ist aber die Verwendung der zuminderst zwei einander diametral gegenüber angeordneten Luftauslasskanäle 27 empfehlenswert. Mehr als zwei unter gleichmässigen Bogenabständen über den Ringkanal 26 verteilte Luftauslasskanäle 27 vermögen diese Wirkung noch zu verbessern.

Vom Ringkanal 26 radial nach innen schliesst an diesen eine die zylinderförmige Öffnung des Druckraums 24 umgreifende kreisförmige Endfläche 28 an. Sie liegt in einer Ebene senkrecht zur Achse. Über die Ebene der Endfläche 28 steht der Wulstring 25 etwas in Richtung auf die Ventilscheibe 2 vor. Damit ist sichergestellt, dass sich die Ventilscheibe auf ihre der Luftauslasshülse 4 zugewandten Seite nur längs der Kreislinie am Scheitel des Wulstrings 25 abstützt.

Der Hülsenschaft 23 weist etwa in seiner Mitte eine flache Ringnut 29 auf, die auf einer Seite von einer in den Druckraum 24 mündenden Radialbohrung 30 durchsetzt ist, deren Zweck unten näher erläutert wird. Zur Fixierung der auf dem Führungsteil 15 verschraubten Luftauslasshülse 4 dient das Fixierstück 6, das im Durchmesser mit dem Führungsteil 15 übereinstimmt und ein Aussengewinde hat, so dass es mit Hilfe eines Schraubenziehers oder dergleichen als Gegenmutter auf die axiale Endfläche des Führungsteils 15 geschraubt und hier festgeklemmt werden kann. Selbstverständlich weist das Fixierstück 6 einen Axialdurchbruch auf, durch den hindurch der Druckraum 24 in zusammengebautem Zustand mit dem Inneren des Druckmesswerkes 1 kommuniziert.

Über die Luftauslasshülse 4 wird aussen eine Dichtung 5 geschoben, die auf der Unterseite des Kopfflansches 22 zur Anlage kommt und von Öffnungen durchbrochen wird, die mit den Luftauslasskanälen 27 fluchten. Die Dichtung 5 wird in dieser Stellung durch den als nächstes über den Hülsenschaft 23 der Luftauslasshülse 4 geschobenen Löffelhalter 7 fixiert. Der Löffelhalter hat die Form einer zylindrischen Hülse, deren Innendurchmesser den Aussendurchmesser des Hülsenschafts 23 der Luftauslasshülse 4 geringfügig übersteigt und deren axiale Länge ebenfalls etwas grösser ist als die axiale Länge des Hülsenschaftes 23. In der der Ventilscheibe 2 zugekehrten Endfläche des Löffelhalters 7 ist ein weiterer Ringkanal 31 vorgesehen, der die Luftauslasskanäle 27 untereinander verbindet und seinerseits durch einen Verbindungskanal 32 an eine mit Innengewinde versehene Radialbohrung 33 angeschlossen ist, die wiederum über die Radialbohrung 30 mit dem Druckraum 24 in Verbindung steht. In die Radialbohrung 33 wird unter Zwischenlage eines Löffels der Anschlusstutzen 14 eingeschraubt, der zum Anschluss einer aufblasbaren Manschette über einen Schlauch dient und im Bereich seines für das Einschrauben in die Radialbohrung 33 bestimmten Gewindezapfens einen Gewindedurchbruch 34 aufweist. Dieser erstreckt sich vertikal zur Achse des Ablassventils. Der Gewindedurchbruch ist so angeordnet, dass er bei eingeschraubtem Anschlusstutzen 14 mit dem Verbindungskanal 32 fluchtet. Der Druckraum 24 ist somit über die Radialbohrung 30, das Innere des Anschlusstutzens 14, den Gewindedurchbruch 34, den Verbindungskanal 32, den Ringkanal 31, die Luftauslasskanäle 27 und den Ringkanal 26 mit dem Raum neben der Ventilscheibe 2 verbunden. Dadurch ist sichergestellt, dass durch die Betätigung der Ventilscheibe 2 tatsächlich eine Verbindung des Druckraums 24 mit der freien Atmosphäre hergestellt werden und der Druck auf diese Weise abgebaut werden kann.

Der in der Figur nicht gezeigte, zwischen Anschlusstutzen 14 und Aussenumfang des Löffelhalters 7 verklemmte Löffel kann überdies durch eine Schraube am Löffelhalter 7 fixiert werden, die unter Zwischenschaltung des Löffels in ein Gewindeloch 35 eingeschraubt wird, das radial in die Wandung des Löffelhalters 7 hinein erstreckt ist und neben der Radialbohrung 33 auf der gleichen achsparallelen Mantellinie liegt.

Das Ablassventil wird dadurch vervollständigt, dass vom noch freien Ende des Löffelhalters 7 her die Teile 8 bis 13 eingesetzt und durch Einschrauben des letztgenannten Teils in das Innengewinde der Luftauslasshülse 4 fixiert werden. Die Funktion des dadurch gebildeten Rückschlagventils 13 versteht sich von selbst. Es dient dazu, bei Betätigung der in der Figur nicht gezeigten Gummiballpumpe durch Abheben des Ventilkonus 11 vom O-Ring 12 den Druckaufbau im Druckraum 24 unter gleichzeitiger Weiterleitung an das Druckmesswerk 1 und (über den Anschlusstutzen 14) an die aufblasbare Manschette zu ermöglichen.

Der Löffelhalter 7 hat bei einer Gesamtlänge von etwa 2 cm einen Durchmesser von etwa 1½ cm. Durch die am Rückschlagventil 13 anschliessende Gummiballpumpe nebst danebenliegendem Löffel ergibt sich so eine ergonomisch günstige Abstützung von Daumen und Zeigefinger des Benutzters am Aussenumfang des Löffelhalters 7. Das gilt sowohl für das reine Ergreifen, wie beim Betätigen des ebenfalls ergonomisch günstig liegendne Auslassringes 3. Herstellungsmässig von besonderem Vorteil ist die kompakte Bauweise aus wenigen auf Automaten preisgünstig zu fertigenden Einzelteilen.

## Patentansprüche

1. Ablassventil für Blutdruckmessgeräte mit einem Ventilgehäuse, dessen Innenraum mit einer aufblasbaren Manschette und einem Druckmesswerk sowie einem Luftauslass in Verbindung steht, der zu einem auf der Aussenseite des Ventilgehäuses angeordneten ebenen Ventilsitz führt und von einer kreisringförmigen, ebenen gummielastischen Ventilscheibe (2) abgedeckt ist, die mit ihrem Zentraldurchbruch (18) koaxial auf einem neben dem Ventilsitz vorgesehenen zapfenförmigen Führungsteil (15) angeordnet ist und an ihrem Aussenrand mit einem koaxialen rotationssymmetrischen Betätigungsglied für das Abheben vom Ventilsitz versehen ist, dadurch gekennzeichnet, dass die Ventilscheibe (2), die nicht von einer Ventilfeder abdichtend beaufschlagt wird, auf ihrer vom Luftauslass abgewandt liegenden Seite in einer ebenen kreisringförmigen Abstützfläche (16) abgestützt ist, deren Aussendurchmesser gleich oder geringfügig grösser als der Durchmesser einer Kreislinie ist, längs der die Ventilscheibe auf der dem Luftauslass zugewandten Seite am Ventilgehäuse abgestützt ist, und dass der Luftauslass einen kleineren Achsabstand als die Kreislinie hat und die Ventilscheibe einen grösseren Aussendurchmesser als die Abstützfläche aufweist.

2. Ablassventil nach Anspruch 1, dadurch gekennzeichnet, dass der Luftauslass Ringform hat und sein Aussendurchmesser nur ganz geringfügig kleiner als der Durchmesser der Kreislinie ist.

3. Ablassventil nach Anspruch 1 oder 2, dadurch gekennzeichnet, dass der Durchmesser der Ventilscheibe (2) mindestens das 1½-fache, vorzugsweise das Doppelte des Durchmessers der Abstützfläche (16) beträgt.

4. Ablassventil nach einem der Ansprüche 1 bis 3, dadurch gekennzeichnet, dass an den Aussenrand der Abstützfläche (16) eine gegenüber dieser um etwa 45° von der Ventilscheibe (2) weggeneigte konische Ringfläche (17) anschliesst.

5. Ablassventil nach einem der Ansprüche 1 bis 4, dadurch gekennzeichnet, dass die Ventilscheibe (2) auf der dem Luftauslass zugewandten Seite von einer Luftauslasshülse (4) abgestützt ist, die kreisförmig um die Achse verteilt eine Vielzahl achsparalleler Luftauslasskanäle (27) aufweist, die in der Ventilscheibe zugewandten Endfläche (28) der Luftauslasshülse innerhalb eines Wulstringes (25) münden, der mit der Ventilscheibe längs der Kreislinie in Berührung steht und eine kreisringförmige Endflächenebene rings umgreift, über die er in Richtung auf die Ventilscheibe (2) geringfügig vorsteht.

6. Ablassventil nach Anspruch 5, dadurch gekennzeichnet, dass die Luftauslasshülse (4) in Abstand von der vom Wulstring (25) umgriffenen Endfläche (28) und parallel zu dieser einen die achsparallelen Luftauslasskanäle (27) verbindenden Ringkanal (31) hat, der auf seiner von der Ventilscheibe abgewandten Seite durch einen ebenfalls achsparallelen Verbindungskanal (32) mit einem zur Achse senkrecht verlaufenden und mit dem Druckraum (24) verbundenen Druckkanal in Verbindung steht.

7. Ablassventil nach Anspruch 6, dadurch gekennzeichnet, dass die Luftauslasshülse (4) im Bereich des Ringkanals (31) in einer Durchmesserstufe auf einen zylindrischen Hülsenschaft (23) verminderten Durchmessers übergeht, auf den eine mit der Luftauslasshülse im Bereich der Durchmesserstufe unter Zwischenlage einer Dichtung (5) den Ringkanal (31) bildende Zylinderhülse (Löffelhalter 7) aufgebracht ist, in die seitlich ein die Zylinderhülse durchsetzender und mit dem Druckraum (24) kommunizierender Anschlussstutzen (14) eingeschraubt ist, in den über eine Öffnung (Gewindedurchbruch 34) der Verbindungskanal (32) mündet.

8. Ablassventil nach einem der Ansprüche 1 bis 7, dadurch gekennzeichnet, dass das ringförmige Betätigungsglied im Querschnitt halbkreisförmig ausgebildet ist, wobei der Halbkreis einen Durchmesser aufweist, der höchstens etwa das Dreifache der Dicke der Ventilscheibe (2) beträgt, mit seiner Bogenlinie von der Achse weg nach aussen weist und auf seiner Innenseite eine Ringnut (20) zur Aufnahme des Aussenrandes der Ventilscheibe hat.

## Claims

1. Discharge valve for an instrument for measuring the blood pressure, with a valve housing whose interior space is connected to an inflatable cuff and a pressure meter, also to an air outlet which leads to a flat valve seat arranged on the outer side of the valve housing and is covered by an annular, flat, elastomeric valve disc (2), the said valve disc being arranged with its central perforation (18) coaxially on a stud-shaped guide portion (15) provided adjacent the valve seat and being provided at its outer edge with a coaxial rotationally symmetrical operating element for lifting away from the valve seat, characterised in that the valve disc (2), which is not acted upon by a valve spring with a sealing effect, is supported at its side remote from the air outlet on a flat annular supporting surface (16) whose outer diameter is equal to or slightly greater than the diameter of a circular line along which the valve disc is supported at the side directed towards the air outlet on the valve housing, and that the air outlet has a smaller axis spacing than the circular line and the valve disc has a larger outer diameter than the supporting surface.

2. Discharge valve according to claim 1, characterised in that the air outlet has an annular shape and its outer diameter is only very slightly smaller than the diameter of the circular line.

3. Discharge valve according to claim 1 or 2, characterised in that the diameter of the valve disc (2) amounts to at least 1½ times, preferably twice, the diameter of the supporting surface (16).

4. Discharge valve according to one of claims 1 to 3, characterised in that there adjoins the outer edge of the supporting surface (16) a conical annular surface (17) which is inclined away from the valve disc (2) at an angle of about 45° relatively to the said supporting surface.

5. Discharge valve according to one of claims 1 to 4, characterised in that the valve disc (2) is supported at the side directed towards the air outlet by an air outlet sleeve (4) comprising a plurality of axially parallel air outlet ducts (27) which are distributed in a circle about the axis and which open into that end face (28) of the air outlet sleeve which is directed towards the valve disc, within an annular bead (25) which is in contact with the valve disc along the circular line and which extends annularly about an annular end face plane beyond which it projects slightly in the direction towards the valve disc (2).

6. Discharge valve according to claim 5, characterised in that the air outlet sleeve (4) comprises, at a distance from the end face (28) surrounded by the annular bead (25) and parallel to this face, an annular duct (31) which connects the axially parallel air outlet ducts (27) and which at its side remote from the valve disc communicates through a likewise axially parallel connecting duct (32) with a pressure duct which extends at right angles to the axis and connects with the pressure chamber (24).

7. Discharge valve according to claim 6, characterised in that the air outlet sleeve (4) is stepped in its diameter to a diameter reduced to a cylindrical sleeve body (23) on to which there is arranged a cylindrical sleeve (spoon holder 7) which with the air outlet sleeve in the region of the diameter step forms the annular duct (31), with interposition of a sealing element (5), and into this latter cylindrical sleeve there is screwed laterally a connecting union (14) which extends through the cylindrical sleeve and which communicates with the pressure chamber (24) and into which the connecting duct (32) opens by way of an opening (screwthread hole 34).

8. Discharge valve according to one of claims 1 to 7, characterised in that the annular operating element is constructed with a semicircular cross-section, the semicircle having a diameter which amounts at the most to about three times the thickness of the valve disc (2), with its arch directed outwardly away from the axis, and having at its inner side an annular groove (20) for receiving the outer edge of the valve disc.

## Revendications

1. Soupape de décharge pour instrument de mesure de la pression artérielle avec un corps de soupape, dont l'espace interne est en communication avec un manchon gonflable et un élément de mesure de la pression ainsi qu'une sortie d'air, qui conduit à un siège plat de soupape disposé sur le côté extérieur du corps de soupape et qui est couvert par un disque plat (2) de forme circulaire et ayant l'élasticité du caoutchouc, qui est disposé, par son ouverture centrale (18) coaxialement sur une pièce de guidage (15) en forme de pivot qui est prévue à proximité du siège de soupape, et qui est pourvu sur son bord externe, d'un organe de mise en action coaxial et symétrique en rotation pour la levée du siège de soupape, caractérisée en ce que le disque (2) qui n'est pas sollicité de manière étanche, par un ressort de soupape, s'appuie, par son côté éloigné de la sortie d'air, sur une surface d'appui (16) plane et de forme circulaire, dont le diamètre externe est égal ou est légèrement plus grand que le diamètre d'une ligne circulaire le long de laquelle le disque s'appuie sur le corps de soupape du côté tourné vers la sortie d'air, et en ce que la sortie d'air a une plus courte distance à l'axe que la ligne circulaire et le disque de soupape présente un plus grand diamètre externe que la surface d'appui.

2. Soupape de décharge selon la revendication 1, caractérisée en ce que la sortie d'air a une forme annulaire et son diamètre externe n'est que légèrement plus petit que le diamètre de la ligne circulaire.

3. Soupape de décharge selon la revendication 1 ou 2, caractérisée en ce que le diamètre du disque (2) représente au moins une fois et demie, avantageusement le double du diamètre de la surface d'appui (16).

4. Soupape de décharge selon l'une quelconque des revendications 1 à 3, caractérisée en ce qu'une surface annulaire conique (17) se raccorde au bord externe de la surface d'appui (16) qui est inclinée de 45° par rapport au disque (2) qui lui fait face.

5. Soupape de décharge selon l'une quelconque des revendications 1 à 4, caractérisée en ce que le disque (2) s'appuie, du côté tourné vers la sortie d'air, sur une douille de sortie d'air (4), qui présente un certain nombre de canaux de sortie d'air (27) parallèles à l'axe, et qui sont répartis en cercle autour de l'axe, qui débouchent dans la surface extrême (28) de la douille de sortie d'air tournée vers le disque, à l'intérieur d'un bourrelet annulaire (25), qui se trouve en contact avec le disque le long de la ligne de cercle et entoure un plan de forme annulaire de la surface extrême au-delà duquel il dépasse légèrement en direction du disque (2).

6. Soupape de décharge selon la revendication 5, caractérisée en ce que la douille de sortie d'air (4) a un canal annulaire (31) reliant les canaux de sortie d'air (27) parallèles à l'axe, à une certaine distance de la surface extrême (28) entourée par le bourrelet annulaire (25) et parallèlement à celle-ci, lequel canal se trouve en communication, par son côté éloigné du disque, au moyen d'un canal de jonction (32) également parallèle à l'axe, avec un canal de pression perpendiculaire à l'axe et relié à l'espace sous pression (24).

7. Soupape de décharge selon la revendication 6, caractérisée en ce que la douille de sortie d'air (4) se transforme, dans la zone du canal annulaire (31), par un diamètre échelonné, en un manchon cylindrique (23) d'un diamètre diminué, où est disposée une douille cylindrique (support de capteur 7) formant avec la douille de sortie d'air, dans la zone de l'échelon de diamètre, avec interposition d'une étanchéité (5), le canal annulaire (31), où est vissé un support de raccordement (14) traversant latéralement le manchon cylindrique et communiquant avec l'espace sous pression (24), dans lequel débouche, par une ouverture (ouverture filetée 34), le canal de jonction (32).

8. Soupape de décharge selon l'une quelconque des revendications 1 à 7, caractérisée en ce que l'organe annulaire de mise en action a une section transversale en demi-cercle, le demi-cercle présentant un diamètre qui atteint, au maximum, à peu près le triple de l'épaisseur du disque (2), sa ligne courbe étant dirigée, de l'axe, vers l'extérieur et il a, sur son côté interne, une rainure annulaire (20) pour la réception du bord externe du disque de la soupape.

FIG.1

FIG. 2